# EUROPEAN PATENT SPECIFICATION

(11) **EP 4 595 987 B1**
(45) Date of publication and mention of the grant of the patent: **13.05.2026**
(21) Application number: 24206294.1
(22) Date of filing: 12.10.2024
(51) Int. Cl.: A61L 9/14

(54) **SPLIT-TYPE MICROPOROUS DIRECT-INJECTION AROMATHERAPY MACHINE**
MIKROPORÖSE AROMATHERAPIEMASCHINE MIT GETEILTER DIREKTEINSPRITZUNG
MACHINE D'AROMATHÉRAPIE MICROPOREUSE À INJECTION DIRECTE DE TYPE DIVISÉE

(30) Priority: 05.02.2024 CN 202410164293
(43) Date of publication of application: 06.08.2025
(73) Proprietor: Foshan Nanhai Keri Electronic Co., Ltd, Foshan City 528200 (CN)
(72) Inventor: WU, Liangxiang, Foshan City, 528200 (CN); LI, Wanlin, Foshan City, 528200 (CN); FENG, Jinyun, Foshan City, 528200 (CN); CHEN, Guoliang, Foshan City, 528200 (CN)
(74) Representative: Murgitroyd & Company

(56) References cited:
- US-A1- 2018 043 048
- US-A1- 2019 298 980
- US-A1- 2022 314 264
- US-B2- 9 027 852

## Description

### TECHNICAL FIELD

The present application relates to the technical field of aromatherapy machines, in particular to a split-type microporous direct injection aromatherapy machine.

### BACKGROUND

Aromatherapy machine is a device that can heat, disperse and release aromatic oil or essential oil, which is widely used in homes, offices, hotels and other places. The aromatherapy machine is usually equipped with an atomizing sheet and a nozzle. The atomizing sheet breaks up the essential oil or sesame oil from a liquid state into a mist, and the mist essential oil is ejected from the nozzle to achieve the effect of releasing fragrance. The shell of the traditional aromatherapy machine is usually an integrated structure, and the atomizing sheet is arranged in the integrated shell. In order to achieve better waterproof, the atomizing sheet is arranged in a sealed electronic control assembly. Because the atomizing sheet may be affected by environmental dust or essential oil impurities, or the essential oil will solidify on the surface of the atomizing sheet after being left idle for a long time, the atomizing sheet is likely to be damaged, which leads to atomization failure. When atomization fails, it is difficult for users to separate and replace the atomizing sheet with an integrated structure. Usually, the whole aromatherapy machine needs to be sent to a professional maintenance center or manufacturer for maintenance or even directly discarded. This structural mode is not conducive to the maintenance of the aromatherapy machine by users and increases the maintenance cost.
A US patent application No. 2022314264A1 discloses a fluid diffuser for atomization of essential oils having a flexible seal used to reduce internal pressure of an air chamber adjacent to the atomizer. It uses a cover and a sensor to control the operation of the atomizer based on the position of the cover. An atomizer housing portion of the diffuser is removable and replaceable relative to a lower portion of the housing.

### SUMMARY

The technical problem to be solved by the present application is to provide a split-type microporous direct-injection aromatherapy machine, in which the atomizing module can be conveniently disassembled, and the overall installation and disassembly operations are simple and convenient.

In order to solve the above technical problems, the present application provides a split-type microporous direct-injection aromatherapy machine, which includes a base, a shell and an atomizing module arranged between the base and the shell, wherein a connecting mechanism is arranged on the base, and the shell and the base are connected with each other through the connecting mechanism. The invention is set out in the appended set of claims.

The base is provided with a slot, and the atomizing module is detachably inserted into the slot; the atomizing module is electrically connected with the base, and the shell can cover a lower part of the atomizing module.

As an improvement of the above solution, the atomizing module includes a liquid storage bottle and a liquid guide tube, and the liquid guide tube is positioned below and communicated with the liquid storage bottle; the base includes an upper mounting stand, the slot is arranged in the upper mounting stand, and the liquid guide tube can be inserted into the slot.

As an improvement of the above solution, the liquid guide tube includes a connecting part and a liquid guide part which are connected with each other; the liquid guide part protrudes from a side of the connecting part, and the connecting part is communicated with the interior of the liquid guide part; an inner wall of the connecting part is provided with an internal thread, a bottle mouth of the liquid storage bottle is provided with an external thread, and the liquid storage bottle is screwed into the connecting part through the external thread.

As an improvement of the above solution, a snap ring is arranged at an upper part of the connecting part, the snap ring protrudes from a side wall of the connecting part and protrudes towards an outer side of the connecting part; an upper surface of the upper mounting stand is provided with an abutting platform, and the bottom of the snap ring is capable of abutting on the abutting platform; an upper inner side of the shell is provided with a pressing plate, and the bottom of the pressing plate abuts on an upper surface of the snap ring.

As an improvement of the above solution, the slot includes a central limit part and a lateral limit part, wherein the position of the central limit part corresponds to that of the connecting part, and the position of the lateral limit part corresponds to that of the liquid guide part; the connecting part can be inserted into the central limit part and the liquid guide part can be inserted into the lateral limit part.

As an improvement of the above solution, the atomizing module further includes an atomizing sheet and an air outlet cover, wherein an accommodating groove is arranged at a tail end of the liquid guide tube, the atomizing sheet is obliquely connected in the accommodating groove and communicated with the interior of the liquid guide tube; a middle part of the air outlet cover is provided with an internal air hole, one side of the internal air hole is communicated with the atomizing sheet, and the other side is communicated with the outside air; an outlet of the outer end of the internal air hole extends obliquely upwards, a lateral part of the air outlet cover is buckled with a side wall of the accommodating groove, and the lateral part of the air outlet cover can cover a lateral part of the atomizing sheet.

As an improvement of the above solution, a lateral part of the shell can cover the liquid guide tube and the air outlet cover; a side wall of the shell is provided with an external air hole, the position of which corresponds to that of the internal air hole, and the external air hole is communicated with the internal air hole; the pressing plate is arranged around the liquid storage bottle, which encloses to form an avoiding hole, and the liquid storage bottle passes through the avoiding hole upwards and is exposed outside the shell.

As an improvement of the above solution, the atomizing module further includes a baffle cover, an electric control board and a first magnetic attraction member; the baffle cover is connected to the bottom of the liquid guide part, an accommodating cavity is arranged in the baffle cover, and the accommodating cavity is communicated with the accommodating groove; the electric control board is arranged in the accommodating cavity and electrically connected with the atomizing sheet, and the bottom of the baffle cover is provided with an electric connection hole; the bottom of the slot is provided with a connecting contact, and the connecting contact can be inserted into the electric connecting hole to be electrically connected with the electric control board; the first magnetic attraction member is arranged in the accommodating cavity, a second magnetic attraction member is arranged in the slot, and the atomizing module is magnetically connected with the slot through the first magnetic attraction member and the second magnetic attraction member.

As an improvement of the above solution, the connecting mechanism includes a buckle block arranged in the shell and a buckle groove arranged on the upper mounting stand; the buckle block is arranged on an inner wall of a lower part of the shell, and the buckle groove is arranged on an outer edge of a lower part of the upper mounting stand; and the buckle block can be inserted into the buckle groove from a lateral part of the buckle groove.

As an improvement of the above solution, a cross section of the upper mounting stand is gradually enlarged from top to bottom; the buckle groove includes an insertion groove and a locking groove, wherein the insertion groove is arranged at a lateral part of the locking groove, and a height of the insertion groove is higher than that of the locking groove; the buckle block can be inserted into the insertion groove from top to bottom and screwed into the locking groove from the insertion groove.

The present application has the following beneficial effects:
The split-type microporous direct-injection aromatherapy machine is provided with a base, a shell and an atomizing module. The atomizing module is arranged between the base and the shell, the shell and the base are connected with each other through the connecting mechanism. The atomizing module is detachably arranged in a slot of the base, and the base supplies power to the atomizing module. When the atomizing module needs to be disassembled, the connecting mechanism is opened first, and then the atomizing module is pulled out after the shell is disassembled, so as to realize the independent disassembly of the atomizing module. Moreover, by using the connecting mechanism, the shell can be quickly installed or disassembled, so that the atomizing module is convenient to install and disassemble, and the whole installation and disassembly operation is simple and convenient.

### BRIEF DESCRIPTION OF DRAWINGS

FIG. 1 is a schematic diagram of the split structure of the split-type microporous direct injection aromatherapy machine of the present application;
FIG. 2 is a schematic sectional view of the split-type microporous direct injection aromatherapy machine of the present application;
FIG. 3 is a schematic view of the atomizing module of the present application in partial section.

### DESCRIPTION OF EMBODIMENTS

In order to make the object, technical solution and advantages of the present application more clear, the present application will be further described in detail with reference to the attached drawings. For this statement, the directional words such as up, down, left, right, front, back, inside and outside that appear or will appear in this paper are only based on the drawings of the present application, and they are not specific limitations on the present application.

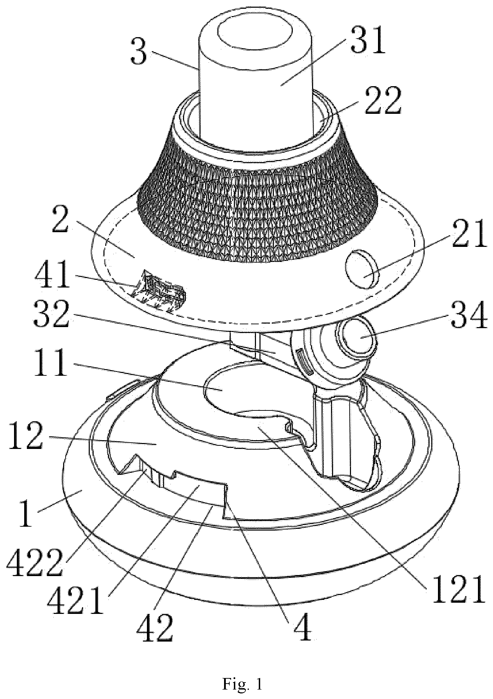

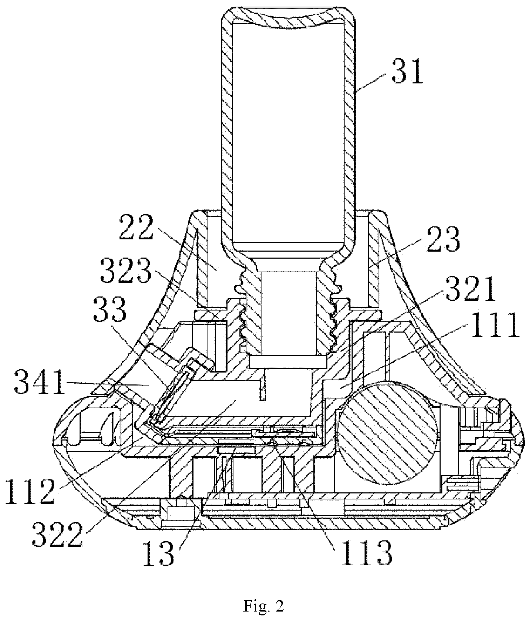

Referring to FIG. 1 and FIG. 2, the embodiment of the present application discloses a split-type microporous direct-injection aromatherapy machine, which includes a base 1, a shell 2 and an atomizing module 3 arranged between the base 1 and the shell 2. A power supply and a control circuit board are arranged in the base 1 for providing power supply and control to the atomizing module 3. A connecting mechanism 4 is arranged on the base 1, and the shell 2 and the base 1 are connected with each other through the connecting mechanism 4, so that by using the connecting mechanism 4, the atomizing module 3 can be quickly realized. With the connecting mechanism 4, the shell 2 and the base 1 can be quickly connected, and the shell 2 can also be quickly detached from the base 1. The base 1 is provided with a slot 11, and the atomizing module 3 can be detachably inserted into the slot 11, so that the atomizing module 3 can be conveniently installed or disassembled by means of plugging, and the use and maintenance are simple. The atomizing module 3 is electrically connected with the base 1, so that the base 1 can supply power to the atomizing module 3. The shell 2 can cover the lower part of the atomizing module 3, and hide the atomizing module 3 while protecting the atomizing module 3, thus improving the integrity of the appearance of the aromatherapy machine.

The embodiment of the present application has the following beneficial effects:
The split-type microporous direct-injection aromatherapy machine of the embodiment of the present application is provided with a base 1, a shell 2 and an atomizing module 3. The atomizing module 3 is arranged between the base 1 and the shell 2, and the shell 2 and the base 1 are connected with each other through the connecting mechanism 4. The atomizing module 3 is detachably arranged in a slot 11 of the base 1, and the base 1 supplies power to the atomizing module 3. When the atomizing module 3 needs to be disassembled, the connecting mechanism 4 is opened first, and then the atomizing module is pulled out after the shell is disassembled, so as to realize the independent disassembly of the atomizing module. Moreover, by using the connecting mechanism, the shell can be quickly installed or disassembled, so that the atomizing module is convenient to install and disassemble, and the whole installation and disassembly operation is simple and convenient.

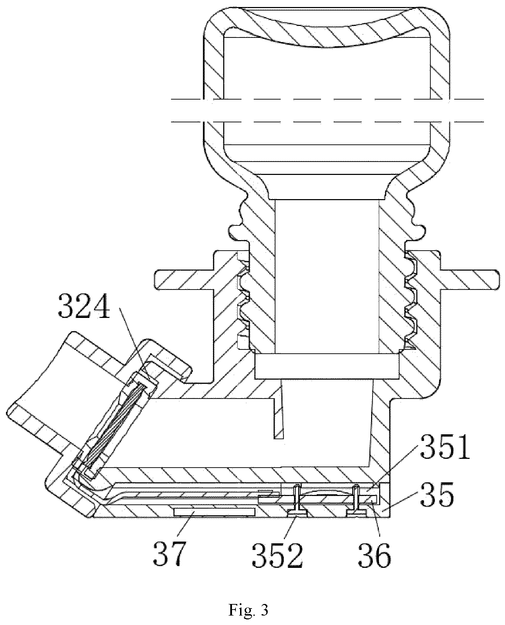

Specifically, referring to FIGS. 2 and 3, the atomizing module 3 includes a liquid storage bottle 31 for storing a liquid such as essential oil, and a liquid guide tube 32 for guiding out the liquid in the liquid storage bottle 31. The liquid guide tube 32 is located below and communicated with the liquid storage bottle 31, and the liquid in the liquid storage bottle 31 can automatically flow into the liquid guide tube 32 under the action of gravity. The base 1 includes an upper mounting stand 12, which is arranged at the upper part of the base 1, and the slot 11 is arranged in the upper mounting stand 12. The liquid guide tube 32 can be inserted into the slot 11 to form a plug-in connection, which is convenient for installation. The middle part of the shell 2 is provided with an avoiding hole 22, and the liquid storage bottle 31 passes upward through the avoidance hole 22 and is exposed outside the shell 2. When disassembling, the whole atomizing module 3 can be taken out by first opening the shell 2 and then grabbing the liquid storage bottle 31 and pulling it upwards, therefore the disassembly is convenient. When installing, the atomizing module 3 is first inserted into the slot 11, and then the shell 2 is covered to make the liquid storage bottle 31 pass through the avoiding hole 22.

Further, the liquid guide tube 32 includes a connecting part 321 and a liquid guide part 322 which are connected with each other. The liquid guide part 322 protrudes from the lateral part of the connecting part 321, and the connecting part 321 is used for forming a connection with the liquid storage bottle 31, so that the liquid guide tube 32 and the liquid storage bottle 31 are connected into a whole. The connecting part 321 is communicated with the interior of the liquid guide part 322. The liquid in the liquid storage bottle 31 flows into the liquid guide part 322 through the connecting part 321. The inner wall of the connecting part 321 is provided with an internal thread, the bottle mouth of the liquid storage bottle 31 is provided with an external thread, and the liquid storage bottle 31 is screwed into the connecting part 321 through the external thread, so that the liquid storage bottle 31 can be conveniently installed and disassembled by using the threaded connection.

In addition, in order to further define the position of the atomizing module 3, a snap ring 323 is arranged at the upper part of the connecting part 321, which protrudes from the side wall of the connecting part 321 and protrudes toward the outside of the connecting part 321. An abutting platform 121 is arranged on the upper surface of the upper mounting stand 12. During installation, when the atomizing module 3 is inserted into the slot 11, the bottom of the snap ring 323 is capable of abutting against the abutting platform, so as to form a limit and facilitate subsequent disassembly. In addition, a pressing plate 23 is arranged on the inner side of the upper part of the shell 2, and the bottom of the pressing plate 23 abuts on the upper surface of the snap ring 323 after the installation, so that the snap ring 323 is tightly fixed up and down.

The slot 11 includes a central limit part 111 and a lateral limit part 112. The position of the central limit part 111 corresponds to that of the connecting part 321, and the position of the lateral limit part 112 corresponds to that of the liquid guide part 322. The central limit part 111 can accommodate and limit the connecting part 321, and the lateral limit part 112 can accommodate and limit the liquid guide part 322. The connecting part 321 can be inserted into the central limit part 111, and the liquid guide part 322 can be inserted into the lateral limit part 112.

The atomizing module 3 further includes an atomizing sheet 33 and an air outlet cover 34. The atomizing sheet 33 atomizes the liquid flowing into the liquid guide part 322. An accommodating groove 324 is arranged at the tail end of the liquid guide tube 32. The atomizing sheet 33 is obliquely connected in the accommodating groove 324 and is communicated with the interior of the liquid guide tube 32. The liquid in the liquid guide tube 32 can infiltrate one side of the atomizing sheet 33. The inclined arrangement of the atomizing sheet 33 can allow the the atomized air to be sprayed upward, so that the atomized air can be more fully dispersed into the surrounding space. The air outlet cover 34 is used for both fixing the atomizing sheet 33 and guiding the atomized air to be ejected. Specifically, the middle part of the air outlet cover 34 is provided with an internal air hole 341, one side of which is communicated with the atomizing sheet 33 and the other side is communicated with the outside air. The outlet of the outer end of the internal air hole 341 extends obliquely upward, so that the atomized air can be guided to be ejected upward. The lateral part of the air outlet cover 34 is buckled with the side wall of the accommodating groove 324, and can cover the lateral part of the atomizing sheet 33, thus limiting and fixing the atomizing sheet 33. The air outlet cover 34 and the accommodating groove 324 are connected by threads. When it is necessary to replace the atomizing sheet 33, after taking out the atomizing module 3, the air outlet cover 34 is disassembled, and then the atomizing sheet 33 is taken out for inspection or replacement.

The lateral part of the shell 2 can cover the liquid guide tube 32 and the air outlet cover 34 to hide the liquid guide tube 32 and the air outlet cover 34, so as to improve the overall aesthetics. The side wall of the shell 2 is provided with an external air hole 21, the position of which corresponds to the position of the internal air hole 341. The external air hole 21 is communicated with the internal air hole 341, and atomized air can be ejected through the external air hole 21. The pressing plate 23 is arranged around the liquid storage bottle 31, and encloses to form an avoiding hole 22. The liquid storage bottle 31 passes through the avoiding hole 22 upward and is exposed outside the shell 2. The pressing plate 23 not only limits the abutting platform 121, but also limits and avoids the liquid storage bottle 31.

The atomizing module 3 further includes a baffle cover 35, an electric control board 36 and a first magnetic attraction member 37. The baffle cover 35 is connected to the bottom of the liquid guide part 322, and is used for sealing the electric control board 36. An accommodating cavity 351 is arranged in the baffle cover 35, and the accommodating cavity 351 is communicated with the accommodating groove 324. The electric control board 36 is arranged in the accommodating cavity 351 and electrically connected with the atomizing sheet 33, and electronic wires are connected between the electric control board 36 of the accommodating cavity 351 and the atomizing sheet 33 of the accommodating groove 324. In order to realize power supply, the bottom of the baffle cover 35 is provided with an electrical connection hole 352, and the bottom of the socket 11 is provided with a connection contact 113. When the atomizing module 3 is inserted, the connection contact 113 can be inserted into the electrical connection hole 352 to be electrically connected with the electric control board 36. The connection contact 113 is electrically connected with the power supply, so that power can be supplied to the atomizing sheet 33 through the electric control board 36.

Further, in order to firmly fix the atomizing module 3, the first magnetic attraction member 37 is arranged in the accommodating cavity 351, and A second magnetic attraction member 13 is arranged in the slot 11. The atomizing module 3 is magnetically connected with the slot 11 through the first magnetic attraction member 37 and the second magnetic attraction member 13. With the magnetic attraction connection, the atomizing module 3 can be quickly connected with the slot 11 during installation, which is simple and labor-saving. The magnetic connection improves the connection stability of the atomizing module 3 and forms a better limit effect.

Referring to FIG. 1, the connecting mechanism 4 includes a buckle block 41 arranged on the shell 2 and a buckle groove 42 arranged on the upper mounting stand 12. The buckle block 41 is arranged on the inner wall of the lower part of the shell 2 and the buckle groove 42 is arranged on the outer edge of the lower part of the upper mounting stand 12. During installation, the shell 2 is buckled outside the liquid storage bottle 31 and abuts against the upper mounting stand 12, and then by rotating, the buckle block 41 can be inserted into the buckle groove 42 from the lateral part of the buckle groove 42. Specifically, the cross section of the upper mounting stand 12 gradually expands from top to bottom to form a truncated cone shape. The buckle groove 42 includes an insertion groove 421 and a locking groove 422. The insertion groove 421 is arranged at the lateral part of the locking groove 422, and the height of the insertion groove 421 is higher than that of the locking groove 422. Therefore, the distance from the edge of the insertion slot 421 to the center of the upper mounting stand 12 is smaller than the distance from the edge of the locking slot 422 to the center of the upper mounting stand 12. The buckle block 41 can be inserted into the insertion slot 421 from top to bottom, and then the buckle block 41 can be screwed into the locking slot 422 from the insertion slot 421 by rotation. The locking slot 422 forms a locking buckle for the buckle block 41, so that the shell 2 can be prevented from moving up and down relative to the base 1, and the user can lift the whole aromatherapy machine by grasping the shell 2, which is convenient to use.

## Claims

1. A split-type microporous direct-injection aromatherapy machine, comprising a base (1), a shell (2) and an atomizing module (3) arranged between the base (1) and the shell (2), wherein a connecting mechanism (4) is arranged on the base (1), and the shell (2) and the base (1) are connected with each other through the connecting mechanism (4); and
the base (1) is provided with a slot (11), and the atomizing module (3) is detachably inserted into the slot (11); the atomizing module (3) is electrically connected with the base (1), and the shell (2) can cover a lower part of the atomizing module (3);
wherein the atomizing module (3) comprises a liquid storage bottle (31) and a liquid guide tube (32), and the liquid guide tube (32) is positioned below and communicated with the liquid storage bottle (31), a liquid in the liquid storage bottle (31) is capable of automatically flowing into the liquid guide tube (32) under an action of gravity; the base (1) comprises an upper mounting stand (12), the slot (11) is arranged in the upper mounting stand (12), and the liquid guide tube (32) can be inserted into the slot (11);
**characterized in that** the connecting mechanism (4) comprises a buckle block (41) arranged in the shell (2) and a buckle groove (42) arranged on the upper mounting stand (12); the buckle block (41) is arranged on an inner wall of a lower part of the shell (2), and the buckle groove (42) is arranged on an outer edge of a lower part of the upper mounting stand (12); and the buckle block (41) is capable of being inserted into the buckle groove (42) from a lateral part of the buckle groove (42).

2. The split-type microporous direct-injection aromatherapy machine according to claim 1, wherein the liquid guide tube (32) comprises a connecting part (321) and a liquid guide part (322) which are connected with each other; the liquid guide part (322) protrudes from a side of the connecting part (321), and the connecting part (321) is communicated with the interior of the liquid guide part (322); an inner wall of the connecting part (321) is provided with an internal thread, a bottle mouth of the liquid storage bottle (31) is provided with an external thread, and the liquid storage bottle (31) is screwed into the connecting part (321) through the external thread.

3. The split-type microporous direct-injection aromatherapy machine according to claim 2, wherein a snap ring (323) is arranged at an upper part of the connecting part (321), the snap ring (323) protrudes from a side wall of the connecting part (321) and protrudes towards an outer side of the connecting part (321); an upper surface of the upper mounting stand (12) is provided with an abutting platform (121), and the bottom of the snap ring (323) is capable of abutting on the abutting platform (121); an upper inner side of the shell (2) is provided with a pressing plate (23), and the bottom of the pressing plate (23) abuts on an upper surface of the snap ring (323).

4. The split-type microporous direct-injection aromatherapy machine according to claim 2, wherein the slot (11) comprises a central limit part (111) and a lateral limit part (112); the position of the central limit part (111) corresponds to that of the connecting part (321), and the position of the lateral limit part (112) corresponds to that of the liquid guide part (322); the connecting part (321) can be inserted into the central limit part (111) and the liquid guide part (322) can be inserted into the lateral limit part (112).

5. The split-type microporous direct-injection aromatherapy machine according to claim 3, wherein the atomizing module (3) further comprises an atomizing sheet (33) and an air outlet cover (34); an accommodating groove (324) is arranged at a tail end of the liquid guide tube (32), the atomizing sheet (33) is obliquely connected in the accommodating groove (324) and communicated with the interior of the liquid guide tube (32); a middle part of the air outlet cover (34) is provided with an internal air hole (341), one side of the internal air hole (341) is communicated with the atomizing sheet (33), and the other side is communicated with the outside air; an outlet of the outer end of the internal air hole (341) extends obliquely upwards, a lateral part of the air outlet cover (34) is buckled with a side wall of the accommodating groove (324), and the lateral part of the air outlet cover (34) can cover a lateral part of the atomizing sheet (33).

6. The split-type microporous direct-injection aromatherapy machine according to claim 5, wherein a lateral part of the shell (2) can cover the liquid guide tube (32) and the air outlet cover (34); a side wall of the shell (2) is provided with an external air hole (21), the position of which corresponds to that of the internal air hole (341), and the external air hole (21) is communicated with the internal air hole (341); the pressing plate (23) is arranged around the liquid storage bottle (31), which encloses to form an avoiding hole (22), and the liquid storage bottle (31) passes through the avoiding hole (22) upwards and is exposed outside the shell (2).

7. The split-type microporous direct-injection aromatherapy machine according to claim 5, wherein the atomizing module (3) further comprises a baffle cover (35), an electric control board (36) and a first magnetic attraction member (37); the baffle cover (35) is connected to the bottom of the liquid guide part (322), an accommodating cavity (351) is arranged in the baffle cover (35), and the accommodating cavity (351) is communicated with the accommodating groove (324); the electric control board (36) is arranged in the accommodating cavity (351) and electrically connected with the atomizing sheet (33), and the bottom of the baffle cover (35) is provided with an electric connection hole; the bottom of the slot (11) is provided with a connecting contact, and the connecting contact can be inserted into the electric connecting hole (352) to be electrically connected with the electric control board (36); the first magnetic attraction member (37) is arranged in the accommodating cavity (351), a second magnetic attraction member (13) is arranged in the slot (11), and the atomizing module (3) is magnetically connected with the slot (11) through the first magnetic attraction member (37) and the second magnetic attraction member (13).

8. The split-type microporous direct-injection aromatherapy machine according to claim 1, wherein a cross section of the upper mounting stand (12) is gradually enlarged from top to bottom; the buckle groove (42) comprises an insertion groove (421) and a locking groove (422); the insertion groove (421) is arranged at a lateral part of the locking groove (422), and a height of the insertion groove (421) is higher than that of the locking groove (422); the buckle block (41) can be inserted into the insertion groove (421) from top to bottom and screwed into the locking groove (422) from the insertion groove (421).

## Patentansprüche

1. Eine geteilte Mikroporen-Direkteinspritzungs-Aromatherapiemaschine, umfassend einen Sockel (1), ein Gehäuse (2), und ein zwischen dem Sockel (1) und dem Gehäuse (2) angeordnetes Verdampfungsmodul (3); wobei ein Verbindungsmechanismus (4) an dem Sockel (1) angeordnet ist, und das Gehäuse (2) und der Sockel (1) durch den Verbindungsmechanismus (4) miteinander verbunden sind; und
wobei der Sockel (1) mit einem Schlitz (11) versehen ist, und das Verdampfungsmodul (3) lösbar in den Schlitz (11) einsetzbar ist; das Verdampfungsmodul (3) elektrisch mit dem Sockel (1) verbunden ist, und das Gehäuse (2) einen unteren Teil des Verdampfungsmoduls (3) abdecken kann;
wobei das Verdampfungsmodul (3) eine Flüssigkeitsspeicherflasche (31) und ein Flüssigkeitsleitrohr (32) umfasst, und das Flüssigkeitsleitrohr (32) unterhalb der Flüssigkeitsspeicherflasche (31) positioniert und mit der Flüssigkeitsspeicherflasche (31) in Verbindung steht, wobei eine Flüssigkeit in der Flüssigkeitsspeicherflasche (31) unter Wirkung der Schwerkraft automatisch in das Flüssigkeitsleitrohr (32) fließen kann; der Sockel (1) einen oberen Montagestand (12) umfasst, der Schlitz (11) in dem oberen Montagestand (12) angeordnet ist, und das Flüssigkeitsleitrohr (32) in den Schlitz (11) eingeführt werden kann;
**dadurch gekennzeichnet, dass** der Verbindungsmechanismus (4) einen Rastblock (41), der in dem Gehäuse (2) angeordnet ist, und eine Rastnut (42), die an dem oberen Montagestand (12) angeordnet ist, umfasst; der Rastblock (41) an einer Innenwand eines unteren Teils des Gehäuses (2) angeordnet ist, und die Rastnut (42) an einem äußeren Rand eines unteren Teils des oberen Montagestands (12) angeordnet ist; und der Rastblock (41) von einem seitlichen Teil der Rastnut (42) in die Rastnut (42) einsetzbar ist.

2. Die geteilte Mikroporen-Direkteinspritzungs-Aromatherapiemaschine nach Anspruch 1, wobei das Flüssigkeitsleitrohr (32) einen Verbindungsteil (321) und einen Flüssigkeitsleitungsteil (322), die miteinander verbunden sind, umfasst; der Flüssigkeitsleitungsteil (322) von einer Seite des Verbindungsteils (321) vorragt, und der Verbindungsteil (321) mit dem Inneren des Flüssigkeitsleitungsteils (322) in Verbindung steht; eine Innenwand des Verbindungsteils (321) mit einem Innengewinde versehen ist, eine Flaschenöffnung der Flüssigkeitsspeicherflasche (31) mit einem Außengewinde versehen ist, und die Flüssigkeitsspeicherflasche (31) durch das Außengewinde in den Verbindungsteil (321) eingeschraubt wird.

3. Die geteilte Mikroporen-Direkteinspritzungs-Aromatherapiemaschine nach Anspruch 2, wobei ein Rastring (323) an einem oberen Teil des Verbindungsteils (321) angeordnet ist; der Rastring (323) von einer Seitenwand des Verbindungsteils (321) vorsteht und zu einer Außenseite des Verbindungsteils (321) hin vorragt; eine obere Fläche des oberen Montagestands (12) mit einer Anlageplattform (121) versehen ist, und der Boden des Rastrings (323) an der Anlageplattform (121) anliegen kann; eine obere Innenseite des Gehäuses (2) mit einer Druckplatte (23) versehen ist, und der Boden der Druckplatte (23) an einer oberen Fläche des Rastrings (323) anliegt.

4. Die geteilte Mikroporen-Direkteinspritzungs-Aromatherapiemaschine nach Anspruch 2, wobei der Schlitz (11) einen zentralen Begrenzungsteil (111) und einen seitlichen Begrenzungsteil (112) umfasst; die Position des zentralen Begrenzungsteils (111) der des Verbindungsteils (321) entspricht, und die Position des seitlichen Begrenzungsteils (112) der des Flüssigkeitsleitungsteils (322) entspricht; der Verbindungsteil (321) in den zentralen Begrenzungsteil (111) eingeführt werden kann, und der Flüssigkeitsleitungsteil (322) in den seitlichen Begrenzungsteil (112) eingeführt werden kann.

5. Die geteilte Mikroporen-Direkteinspritzungs-Aromatherapiemaschine nach Anspruch 3, wobei das Verdampfungsmodul (3) ferner ein Verdampfungsplättchen (33) und eine Luftauslassabdeckung (34) umfasst; eine Aufnahmefuge (324) an einem Endstück des Flüssigkeitsleitrohrs (32) angeordnet ist, das Verdampfungsplättchen (33) schräg in der Aufnahmefuge (324) verbunden und mit dem Inneren des Flüssigkeitsleitrohrs (32) in Verbindung ist; ein Mittelteil der Luftauslassabdeckung (34) mit einem inneren Luftloch (341) versehen ist, eine Seite des inneren Luftlochs (341) mit dem Verdampfungsplättchen (33) in Verbindung steht, und die andere Seite mit der Außenluft in Verbindung steht; ein Auslass des äußeren Endes des inneren Luftlochs (341) sich schräg nach oben erstreckt, eine seitliche Seite der Luftauslassabdeckung (34) mit einer Seitenwand der Aufnahmefuge (324) verrastet ist, und die seitliche Seite der Luftauslassabdeckung (34) eine seitliche Seite des Verdampfungsplättchens (33) abdecken kann.

6. Die geteilte Mikroporen-Direkteinspritzungs-Aromatherapiemaschine nach Anspruch 5, wobei ein seitlicher Teil des Gehäuses (2) das Flüssigkeitsleitrohr (32) und die Luftauslassabdeckung (34) abdecken kann; eine Seitenwand des Gehäuses (2) mit einem äußeren Luftloch (21) versehen ist, dessen Position der des inneren Luftlochs (341) entspricht, und das äußere Luftloch (21) mit dem inneren Luftloch (341) in Verbindung steht; die Druckplatte (23) um die Flüssigkeitsspeicherflasche (31) herum angeordnet ist, die ein Aussparungsloch (22) umschließt, und die Flüssigkeitsspeicherflasche (31) durch das Aussparungsloch (22) nach oben verläuft und außerhalb des Gehäuses (2) freigelegt ist.

7. Die geteilte Mikroporen-Direkteinspritzungs-Aromatherapiemaschine nach Anspruch 5, wobei das Verdampfungsmodul (3) ferner eine Prallabdeckung (35), eine elektrische Steuerplatine (36), und ein erstes Magnetglied (37) umfasst; die Prallabdeckung (35) mit dem Boden des Flüssigkeitsleitungsteils (322) verbunden ist, ein Aufnahmeverk (351) in der Prallabdeckung (35) angeordnet ist, und das Aufnahmeverk (351) mit der Aufnahmefuge (324) in Verbindung steht; die elektrische Steuerplatine (36) in dem Aufnahmeverk (351) angeordnet und elektrisch mit dem Verdampfungsplättchen (33) verbunden ist, und der Boden der Prallabdeckung (35) mit einem elektrischen Verbindungsloch versehen ist; der Boden des Schlitzes (11) mit einem Verbindungskontakt versehen ist, und der Verbindungskontakt in das elektrische Verbindungsloch (352) eingeführt werden kann, um mit der elektrischen Steuerplatine (36) elektrisch verbunden zu werden; das erste Magnetglied (37) in dem Aufnahmeverk (351) angeordnet ist, ein zweites Magnetglied (13) in dem Schlitz (11) angeordnet ist, und das Verdampfungsmodul (3) durch das erste Magnetglied (37) und das zweite Magnetglied (13) magnetisch mit dem Schlitz (11) verbunden ist.

8. Die geteilte Mikroporen-Direkteinspritzungs-Aromatherapiemaschine nach Anspruch 1, wobei ein Querschnitt des oberen Montagestands (12) von oben nach unten allmählich erweitert ist; die Rastnut (42) eine Einführnut (421) und eine Verriegelungsnut (422) umfasst; die Einführnut (421) an einer seitlichen Seite der Verriegelungsnut (422) angeordnet ist, und eine Höhe der Einführnut (421) höher ist als die der Verriegelungsnut (422); der Rastblock (41) von oben nach unten in die Einführnut (421) eingeführt und von der Einführnut (421) in die Verriegelungsnut (422) eingeschwenkt werden kann.

## Revendications

1. Machine d'aromathérapie microporeuse à injection directe de type divisée, comprenant une base (1), une coque (2) et un module d'atomisation (3) disposé entre la base (1) et la coque (2), dans laquelle un mécanisme de connexion (4) est disposé sur la base (1), et la coque (2) et la base (1) se relient l'une à l'autre par l'intermédiaire du mécanisme de connexion (4) ; et
la base (1) est pourvue d'une fente (11), et le module d'atomisation (3) est inséré de manière amovible dans la fente (11) ; le module d'atomisation (3) est connecté électriquement à la base (1), et la coque (2) est configurée pour recouvrir une partie inférieure du module d'atomisation (3) ;
dans laquelle le module d'atomisation (3) comprend un flacon de stockage de liquide (31) et un tube de guidage de liquide (32), et le tube de guidage de liquide (32) est positionné en dessous et communiqué avec le flacon de stockage de liquide (31), un liquide dans le flacon de stockage de liquide (31) est configuré pour s'écouler automatiquement jusqu'à l'intérieur du tube de guidage de liquide (32) sous l'action de la gravité ; la base (1) comprend un support de montage supérieur (12), la fente (11) est disposée dans le support de montage supérieur (12), et le tube de guidage de liquide (32) est configuré pour être inséré dans la fente (11) ;
**caractérisée en ce que** le mécanisme de connexion (4) comprend un bloc d'encliquetage (41) disposé dans la coque (2) et une rainure d'encliquetage (42) disposée sur le support de montage supérieur (12) ; le bloc d'encliquetage (41) est disposé sur une paroi interne d'une partie inférieure de la coque (2), et la rainure d'encliquetage (42) est disposée sur un bord externe d'une partie inférieure du support de montage supérieur (12) ; et le bloc d'encliquetage (41) est configuré pour être inséré dans la rainure d'encliquetage (42) à partir d'une partie latérale de la rainure d'encliquetage (42).

2. Machine d'aromathérapie microporeuse à injection directe de type divisée selon la revendication 1, dans laquelle le tube de guidage de liquide (32) comprend une partie de connexion (321) et une partie de guidage de liquide (322) qui se relient l'une à l'autre ; la partie de guidage de liquide (322) fait saillie à partir d'un côté de la partie de connexion (321), et la partie de connexion (321) est communiquée avec l'intérieur de la partie de guidage de liquide (322) ; une paroi interne de la partie de connexion (321) est pourvue d'un filetage intérieur, une bouche de flacon du flacon de stockage de liquide (31) est pourvue d'un filetage extérieur, et le flacon de stockage de liquide (31) est vissée jusqu'à l'intérieur de la partie de connexion (321) à travers le filetage extérieur.

3. Machine d'aromathérapie microporeuse à injection directe de type divisée selon la revendication 2, dans laquelle un anneau d'enclenchement (323) est disposé à une partie supérieure de la partie de connexion (321), l'anneau d'enclenchement (323) fait saillie à partir d'une paroi latérale de la partie de connexion (321) et fait saillie vers un côté externe de la partie de connexion (321) ; une surface supérieure du support de montage supérieur (12) est pourvue d'une plate-forme d'appui (121), et le fond de l'anneau d'enclenchement (323) est configuré pour s'appuyer sur la plate-forme d'appui (121) ; un côté interne supérieure intérieure de la coque (2) est pourvu d'une plaque de pression (23), et le fond de la plaque de pression (23) s'appuie sur une surface supérieure de l'anneau d'enclenchement (323).

4. Machine d'aromathérapie microporeuse à injection directe de type divisée selon la revendication 2, dans laquelle la fente (11) comprend une partie de limitation centrale (111) et une partie de limitation latérale (112) ; la position de la partie de limitation centrale (111) correspond à celle de la partie de connexion (321), et la position de la partie de limitation latérale (112) correspond à celle de la partie de guidage de liquide (322) ; la partie de connexion (321) est configurée pour être insérée dans la partie de limitation centrale (111) et la partie de guidage de liquide (322) est configurée pour être insérée dans la partie de limitation latérale (112).

5. Machine d'aromathérapie microporeuse à injection directe de type divisée selon la revendication 3, dans laquelle le module de atomisation (3) comprend en outre une feuille d'atomisation (33) et un couvercle de sortie d'air (34) ; une rainure d'accueil (324) est disposée à une extrémité arrière du tube de guidage de liquide (32), la feuille d'atomisation (33) est connectée obliquement dans la rainure d'accueil (324) et communiquée avec l'intérieur du tube de guidage de liquide (32) ; une partie médiane du couvercle de sortie d'air (34) est pourvue d'un trou d'air intérieur (341), un côté du trou d'air intérieur (341) est communiqué avec la feuille d'atomisation (33), et l'autre côté est communiqué avec l'air extérieur ; une sortie de l'extrémité extérieure du trou d'air intérieur (341) s'étend obliquement vers le haut, une partie latérale du couvercle de sortie d'air (34) est encliquetée avec une paroi latérale de la rainure d'accueil (324), et la partie latérale du couvercle de sortie d'air (34) est configurée pour recouvrir une partie latérale de la feuille d'atomisation (33).

6. Machine d'aromathérapie microporeuse à injection directe de type divisée selon la revendication 5, dans laquelle une partie latérale de la coque (2) est configurée pour recouvrir le tube de guidage de liquide (32) et le couvercle de sortie d'air (34) ; une paroi latérale de la coque (2) est pourvue d'un trou d'air extérieur (21), dont la position correspond à celle du trou d'air intérieur (341), et le trou d'air extérieur (21) est communiqué avec le trou d'air intérieur (341) ; la plaque de pression (23) est disposée autour du flacon de stockage de liquide (31), qui enferme pour former un trou d'évitement (22), et le flacon de stockage de liquide (31) passe à travers le trou d'évitement (22) vers le haut et est exposé à l'extérieur de la coque (2).

7. Machine d'aromathérapie microporeuse à injection directe de type divisée selon la revendication 5, dans laquelle le module d'atomisation (3) comprend en outre un couvercle à déflecteur (35), une carte de commande électrique (36) et un premier élément d'attraction magnétique (37) ; le couvercle à déflecteur (35) est connecté au fond de la partie de guidage de liquide (322), une cavité d'accueil (351) est disposée dans le couvercle à déflecteur (35), et la cavité d'accueil (351) est communiquée avec la rainure d'accueil (324) ; la carte de commande électrique (36) est disposée dans la cavité d'accueil (351) et est connectée électriquement à la feuille d'atomisation (33), et le fond du couvercle à déflecteur (35) est pourvu d'un trou de connexion électrique ; le fond de la fente (11) est pourvu d'un contact de connexion, et le contact de connexion est configuré pour être inséré dans le trou de connexion électrique (352) pour être connecté électriquement à la carte de commande électrique (36) ; le premier élément d'attraction magnétique (37) est disposé dans la cavité d'accueil (351), un second élément d'attraction magnétique (13) est disposé dans la fente (11), et le module d'atomisation (3) est connecté magnétiquement à la fente (11) par l'intermédiaire du premier élément d'attraction magnétique (37) et du second élément d'attraction magnétique (13).

8. Machine d'aromathérapie microporeuse à injection directe de type divisée selon la revendication 1, dans laquelle une section transversale du support de montage supérieur (12) s'élargit progressivement de haut en bas ; la rainure d'encliquetage (42) comprend une rainure d'insertion (421) et une rainure de verrouillage (422) ; la rainure d'insertion (421) est disposée sur une partie latérale de la rainure de verrouillage (422), et une hauteur de la rainure d'insertion (421) est supérieure à celle de la rainure de verrouillage (422) ; le bloc d'encliquetage (41) est configuré pour être inséré dans la rainure d'insertion (421) de haut en bas et vissé dans la rainure de verrouillage (422) à partir de la rainure d'insertion (421).
